# EUROPEAN PATENT APPLICATION

(11) **EP 3 388 083 A1**
(43) Date of publication of application: **17.10.2018**
(21) Application number: 18153044.5
(22) Date of filing: 11.03.2013
(51) Int. Cl.: A61K 39/395, C07K 16/18, C07K 16/28, A61P 35/00

(54) **COMPOSITIONS AND METHODS FOR TREATING CANCER**

(30) Priority: 09.03.2012 US 201261608971 P
(62) Divisional of application: 13758403.3
(71) Applicant: Lankenau Institute for Medical Research, Wynnewood, PA 19096 (US)
(72) Inventor: George-Weinstein, Mindy, Wynnewood, PA 19096 (US); Gilmour, Susan, Wynnewood, PA 19096 (US); Gerhart, Jacquelyn, Wynnewood, PA 19096 (US)
(74) Representative: Potter Clarkson LLP

(57) **Abstract**

A method of inhibiting cancer in a subject in need thereof, said method comprising administering to the subject a therapeutically effective amount of at least one Myo/Nog cell targeting molecule and, optionally, at least one cytotoxic molecule.

A method of detecting an increase risk for cancer or metastatic potential in a subject, said method comprising detecting Myo/Nog cells in a biological sample obtained from a subject, wherein an increase in Myo/Nog cells in the biological sample compared to a corresponding biological sample in a normal subject is indicative of an increased risk of cancer or metastatic potential in said subject.

## Description

This application claims priority to U.S. Application Serial No. 61/608,971, filed March 9, 2012, which is hereby incorporated by reference in its entirety.

This invention was made with government support under Grant No. AR052326 and CA070739 awarded by the National Institutes of Health. The Government has certain rights in this invention.

### FIELD OF THE INVENTION

The present invention relates to the field of cancer therapy. Specifically, compositions and methods for detecting, prognosing, inhibiting, treating, and/or preventing cancer are disclosed.

### BACKGROUND OF THE INVENTION

Several publications and patent documents are cited throughout the specification in order to describe the state of the art to which this invention pertains. Each of these citations is incorporated herein by reference as though set forth in full.

Most current forms of chemotherapy target the tumor cells themselves. However, cells within the tumor stroma play a role in tumor progression. Accordingly, agents that target these cells may be useful components of a therapeutic regimen to reduce tumor burden.

### SUMMARY OF THE INVENTION

In accordance with the present invention, methods of inhibiting, treating, and/or preventing cancer in a subject are provided. In certain embodiments, the methods comprise administering to the subject a therapeutically effective amount of at least one Myo/Nog cell targeting molecule and at least one cytotoxic molecule. The Myo/Nog cell targeting molecule may be conjugated to the cytotoxic molecule(s). In a particular embodiment, the method further comprises at least one other anti-cancer measure, such as the administration of at least one other chemotherapeutic agent, tumor excision, and/or administration of radiation therapy.

In accordance with another aspect of the instant invention, methods of detecting an increased risk, diagnosing, or providing a prognosis for cancer or metastatic potential in a subject are provided. In certain embodiments, the method comprises detecting Myo/Nog cells in a biological sample obtained from a subject, wherein an increase in Myo/Nog cells in the biological sample and/or localization compared to a corresponding biological sample in a normal subject is indicative of an increased risk of cancer or metastatic potential in the test subject. In a particular embodiment, the method comprises contacting the biological sample with at least one Myo/Nog cell targeting molecule conjugated to at least one detection molecule.

### BRIEF DESCRIPTIONS OF THE DRAWING

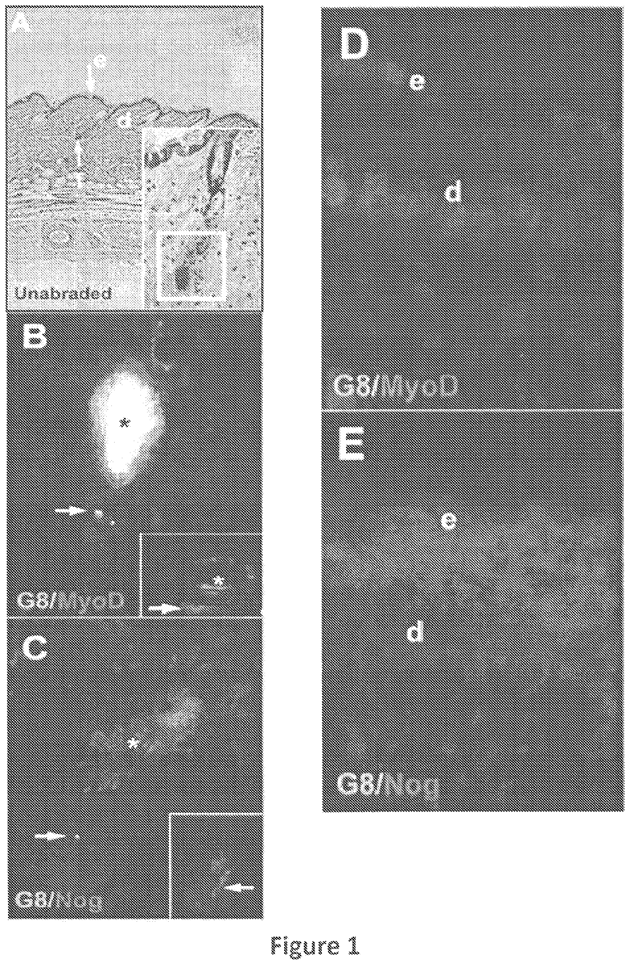

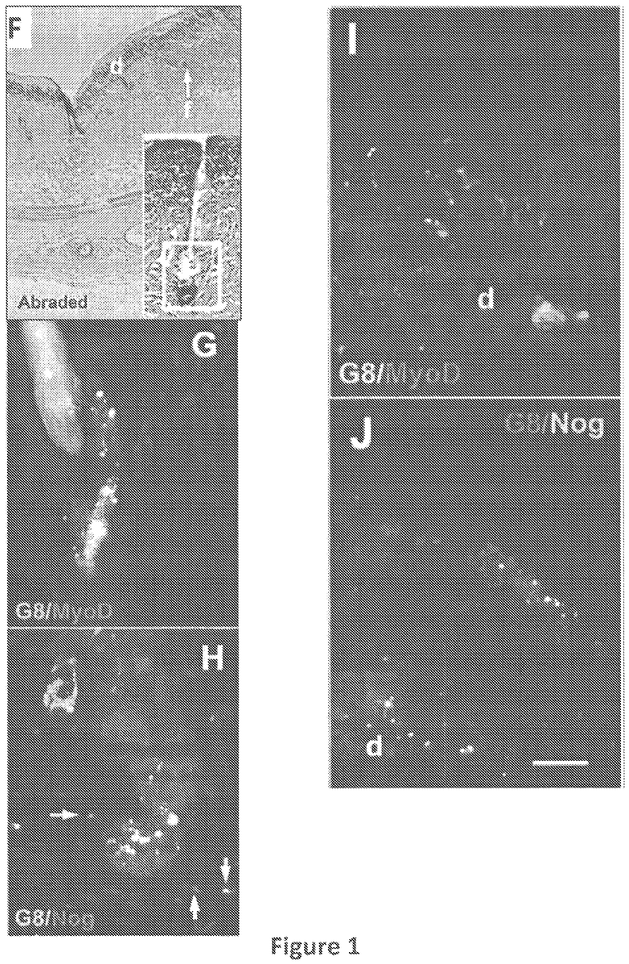

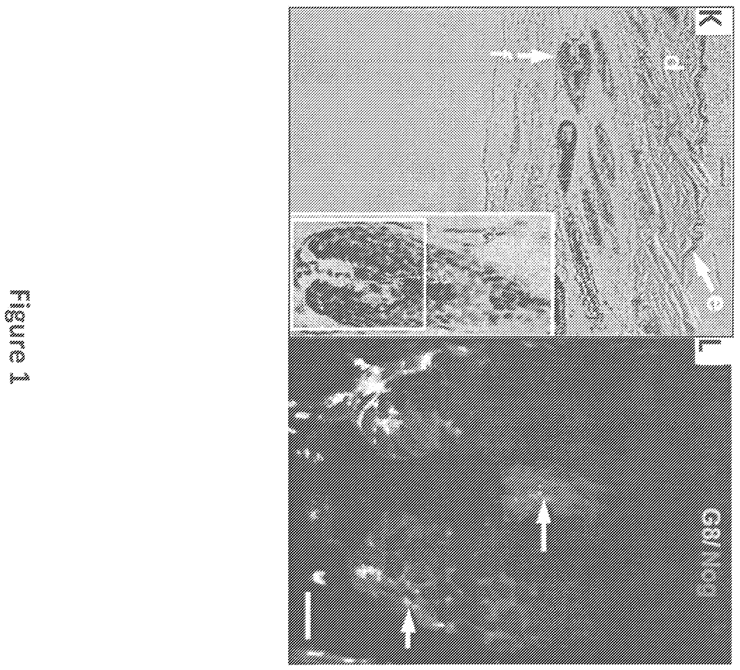

Figures 1 provides images of Myo/Nog cells in normal and abraded murine skin. Skin sections were stained with H&E (Figs. 1A and 1F) or double labeled for G8 and MyoD mRNA, noggin mRNA (Nog) or noggin protein (inset in Fig. 1C). Nuclei were stained with Hoechst dye. The hair shaft is auto-fluorescent (*). In unabraded skin, Myo/Nog cells were located in the hair bulb of the follicle (f) (Figs. 1B and 1C). Inset in Fig. 1B shows Myo/Nog cells within a follicle sectioned transversely. Myo/Nog cells were not observed in the interfollicular dermis (d) or epidermis (e) (Figs. 1B-1E). Following abrasion, Myo/Nog cells were increased in the follicles and surrounding dermis (Figs. 1G and 1H) and were present at the wounded surface (Figs. 1I and 1J). Figures 1K (stained with H&E) and 1L (double labeled for G8 and noggin mRNA) provide images of anagen phase skin. Insets in Figs. 1A and 1F illustrate the areas shown at high magnification in the fluorescence photomicrographs. Bar = 56 µm in Figs. 1A, 1F, and 1K and 9 µm in Figs. 1B-1E, 1G-1J, and 1L.

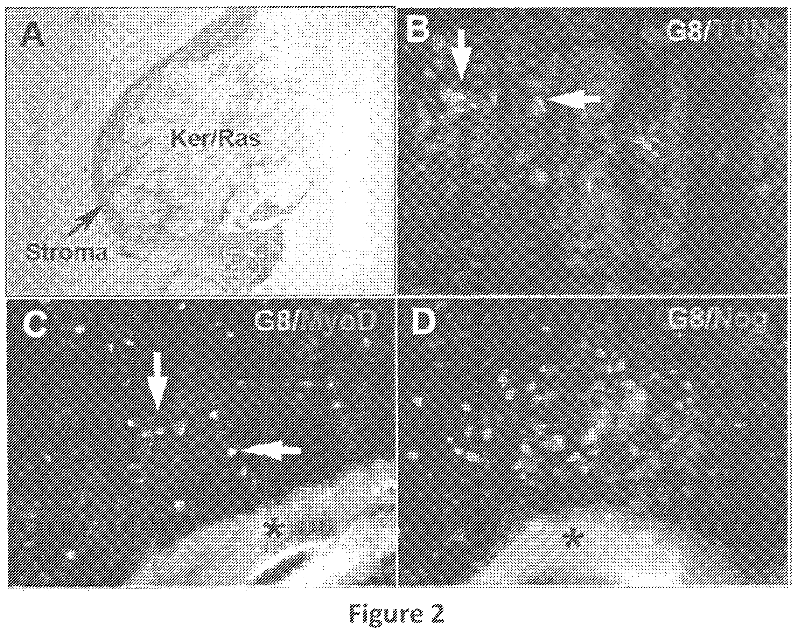

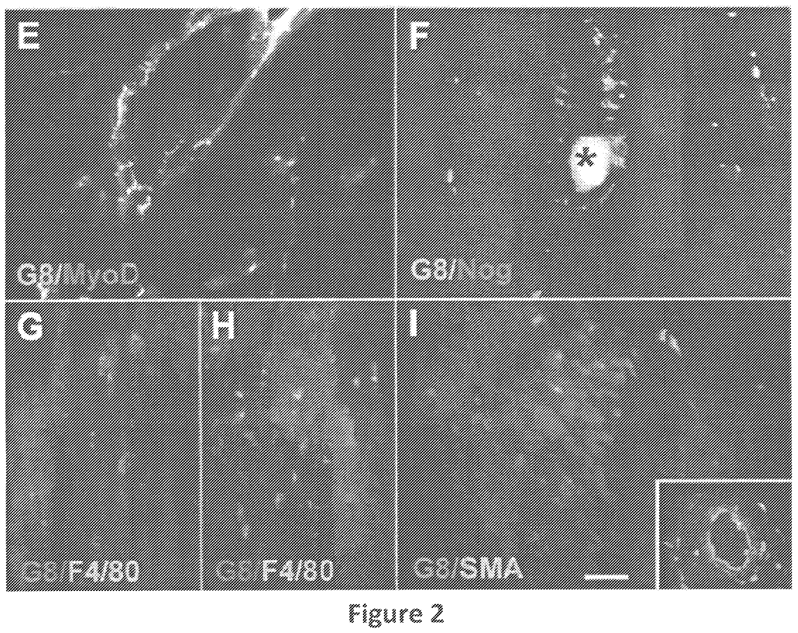

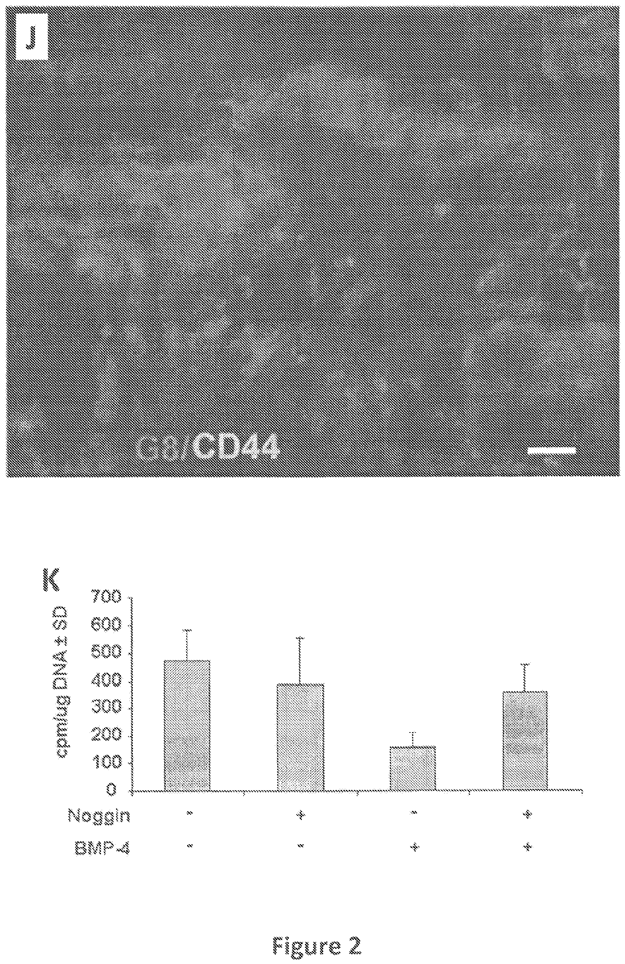

Figure 2 provides images of Myo/Nog cells, noggin and murine carcinoma cells. Murine skin sections containing Ker/Ras tumors were stained with H&E (Fig. 2A) or double labeled for G8 and TUNEL, MyoD mRNA, noggin mRNA (Nog), F4/80 or α-SMA. Keratin within Ker/Ras cells and hair shaft is autofluorescent (*). Myo/Nog cells were present in the tumor stroma (Figs. 2B-2D and 2G-2I). Some Myo/Nog cells were adjacent to TUNEL+ cells (Fig. 2B). Hair follicles overlying the Ker/Ras tumor contained increased numbers of Myo/Nog cells (Figs. 2E and 2F). Antibodies to G8, F4/80, α-SMA, and CD44 labeled separate cells in the stroma (Figs. 2G-2J). Inset in Fig. 2I shows a blood vessel stained with α-SMA. Bar = 135 µm in Fig. 2A and 9 µm in Figs. 2B-J. Cultured Ker/Ras cells were treated with BMP-4 and/or noggin then incubated with [³H]Thymidine (Fig. 2K). Noggin reversed BMP-4's inhibition of [³H]Thymidine incorporation into DNA.

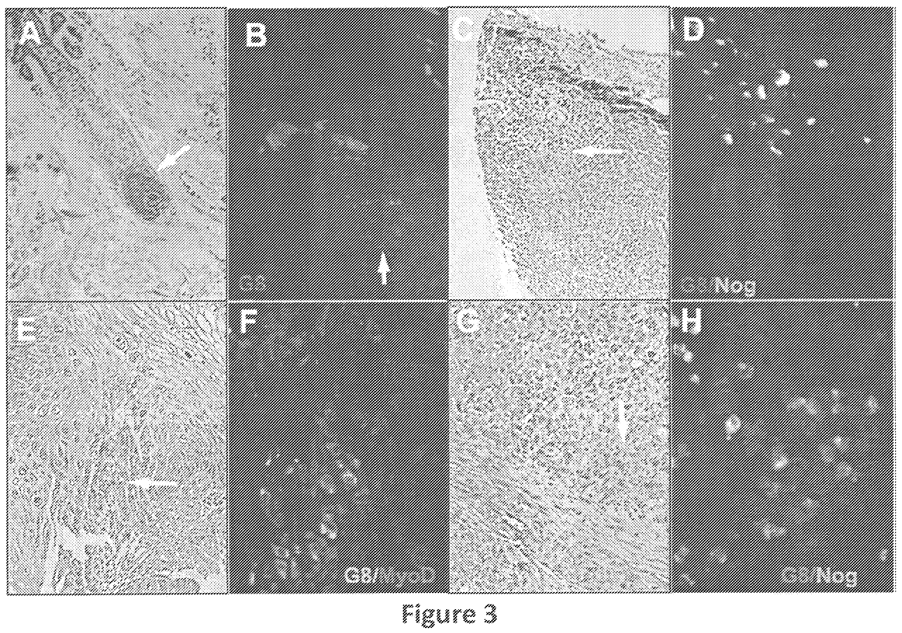

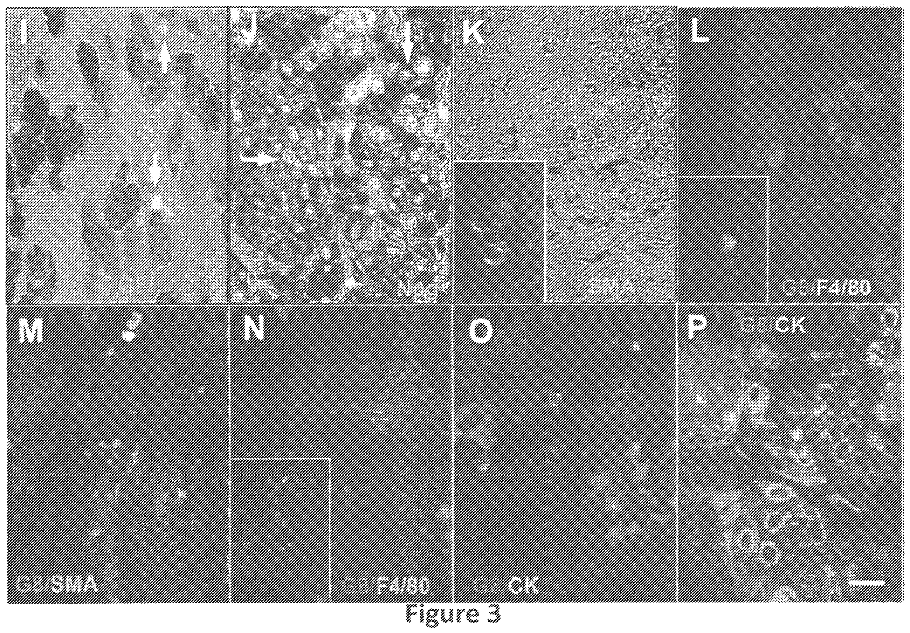

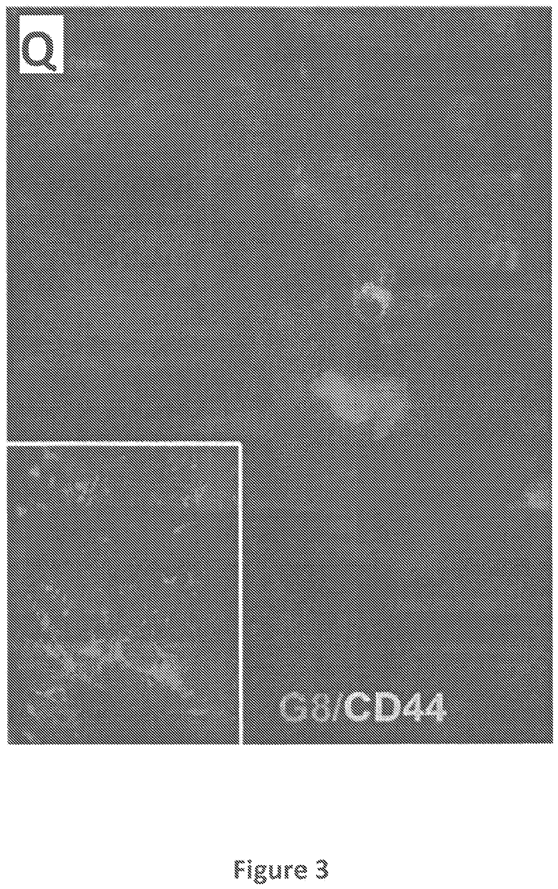

Figure 3 provides images of Myo/Nog cells in normal human skin and tumors. Sections of normal dermis (Figs. 3A and 3B), basal cell carcinoma (Figs. 3C and 3D), squamous cell carcinoma (Figs. 3E-3H and 3M-3P) and malignant melanoma (Figs. 3I-3L) were stained with H&E or double labeled for G8 and MyoD mRNA, noggin protein (Nog), F4/80, α-SMA or cytokeratin (CK). Arrows in H&E stained sections indicate the approximate areas shown in high magnification fluorescent images. Figs. 3I-3K are merges of DIC and fluorescent images. Myo/Nog cells were present in the hair bulb and dermal papillae (arrow) (Fig. 3B), and beneath basal cell carcinoma cells (Fig. 3D). In squamous cell carcinoma, Myo/Nog cells were present in the stroma (Fig. 3F) and among the cancer cells (Fig. 3H). Myo/Nog cells surrounded pigmented melanoma cells (arrows in Fig. 3I). Some Myo/Nog cells were pigmented (arrows in Fig. 3J). Antibodies to G8, F4/80, α-SMA, cytokeratin (CK), and CD44 labeled separate cells in tumor tissue (Figs. 3K-3Q). Insets in Fig. 3L and Fig. 3M: cells labeled for F4/80 but not G8. Bar = 27 µm in Figs. 3A, 3C, 3E, 3G and 9 µm in Figs. 3B, 3D, 3F, 3H-3P.
Figure 4 provides images of Myo/Nog cells expressing the Pax3/FOXO1 translocation in a genetically engineered mouse model of alveolar rhabdomyosarcoma (ARMS). Arrows indicate cells co-expressing the translocation and G8. Inset shows the double labeling for G8 and noggin.
Figure 5 provides an image of human embryonal and alveolar rhabdomyosarcoma cells (ERMS and ARMS, respectively) stained with antibodies to G8 and noggin.
Figure 6 shows the targeting of Myo/Nog cells in cultures of ERMS and ARMS cells. ERMS and ARMS cultures were incubated with the G8 antibody (Figs. 6A and 6D), complement (Figs. 6B and 6E), or both (Figs. 6C and 6F). The cells were then labeled for G8 and TUNEL.
Figure 7 provides images of sections of human osteosarcoma, Wilms and rhabdomyosarcoma tumors labeled with antibodies to G8 and noggin. G8+/noggin+ cells were a subpopulation within the tumors.
Figure 8 provides a list of target epitopes. From top to bottom, the sequences provided are SEQ ID NOs: 6-33.

### DETAILED DESCRIPTION OF THE INVENTION

Bone morphogenetic proteins (BMPs) are powerful regulators of proliferation, differentiation and apoptosis in many embryonic and adult tissues (Bragdon et al. (2011) Cell Signal 23:609-620). In postnatal skin, BMP signaling plays a key role in controlling normal epidermal homeostasis, hair follicle growth and melanogenesis (Botchkarev, V.A. (2003) J. Invest. Dermatol., 120:36-47; Owens et al. (2008) J. Invest. Dermatol., 128:783-790). Targeted knockout of the BMP receptor 1A (BMPR-1A) in the skin inhibits the differentiation of cells that produce the hair shaft (Yuhki et al. (2004) Development 131:1825-1833; Zhang et al. (2006) Stem Cells, 24:2826-2839; Kobielak et al. (2007) Proc. Natl. Acad. Sci., 104:10063-10068; Andl et al. (2004) Development 131:2257-2268; Ming et al. (2004) Genesis 39:10-25). BMPs are also involved in tissue remodeling processes such as wound healing, psoriasis and tumorigenesis (Ming et al. (2004) Genesis 39:10-25; Kaiser et al. (1998) J. Invest. Dermatol., 111:1145-1152; Blessing et al. (1996) J. Cell Biol., 135:227-239; Blessing et al. (1995) Teratog. Carcinog. Mutagen., 15:11-21). BMP-6 is elevated in keratinocytes adjacent to sites of hyperproliferation and migration during normal wound healing and is abnormally expressed throughout all suprabasal layers of chronic wounds and psoriatic lesions (Kaiser et al. (1998) J. Invest. Dermatol., 111:1145-1152; Wall et al. (1993) J. Cell Biol., 120:493-502). Overexpression of BMP-6 in the skin significantly delays wound healing (Kaiser et al. (1998) J. Invest. Dermatol., 111:1145-1152). Moreover, BMP-6 represses epidermal cell proliferation and the formation of carcinogen-induced skin tumors (Blessing et al. (1996) J. Cell Biol., 135:227-239; Drozdoff et al. (1994) Proc. Natl. Acad. Sci., 91:5528-5532; Wach et al. (2001) Oncogene 20:7761-7769), while inhibition of BMP signaling via knockout of BMPR-1A or Smad4 results in the formation of tumors derived from hair follicle cells (Ming et al. (2004) Genesis 39:10-25; Blessing et al. (1996) J. Cell Biol., 135:227-239; Blessing et al. (1995) Teratog. Carcinog. Mutagen., 15:11-21; Qiao et al. (2006) Oncogene 25:207-217; Yang et al. (2005) Cancer Res., 65:8671-8678)

The activity of BMPs is regulated, in part, by a number of secreted BMP antagonists, including noggin, chordin, follistatin, and Cerberus/DAN/gremlin (Bragdon et al. (2011) Cell Signal 23:609-620; Walsh et al. (2010) Trends Cell Biol., 20:244-256). Noggin is expressed in dermal papillae and follicles in anagen phase skin where it promotes new hair growth (Botchkarev et al. (2001) FASEB J., 15:2205-2214). Mice lacking noggin display impaired hair follicle induction and morphogenesis (Botchkarev et al. (2001) FASEB J., 15:2205-2214; Jamora et al. (2003) Nature 422:317-322). However, titration of BMP signaling must be tightly regulated as increased activity of BMP inhibitors also disrupts tissue homeostasis. Overexpression of noggin in the skin results in the spontaneous development of trichofolliculoma-like tumors (Sharov et al. (2009) Am. J. Pathol., 175:1303-1314). Gremlin promotes the proliferation of basal cell carcinoma cells whose growth is inhibited by BMP (Sneddon et al. (2006) Proc. Natl. Acad. Sci., 103:14842-14847). In all, these studies indicate that local modulation of BMP signaling affects hair follicle development and maintenance, epidermal wound repair and skin tumorigenesis.

A cell that modulates BMP signaling and participates in wound healing in the chick embryo has been discovered (Gerhart et al. (2011) Dev. Biol., 359:12-25; Gerhart et al. (2006) J. Cell Biol., 175:283-292; Walker et al. (2010) Proc. Natl. Acad. Sci,. 107:13730-13735; Gerhart et al. (2009) Dev. Biol., 336:30-41; Gerhart et al. (2004) J. Cell Biol., 164:739-746). "Myo/Nog" cells were identified by their expression of mRNA for the skeletal muscle specific transcription factor MyoD, noggin and the G8 cell surface antigen (Gerhart et al. (2011) Dev. Biol., 359:12-25; Gerhart et al. (2006) J. Cell Biol., 175:283-292; Gerhart et al. (2009) Dev. Biol., 336:30-41; Gerhart et al. (2001) J. Cell Biol., 155:381-392; Gerhart et al. (2000) J. Cell Biol., 149:825-834; Strony et al. (2005) Gene Expr. Patterns 5:387-395). Myo/Nog cells emerge in the epiblast and are incorporated into a variety of tissues derived from all three germ layers (Gerhart et al. (2011) Dev. Biol., 359:12-25; Gerhart et al. (2006) J. Cell Biol., 175:283-292; Gerhart et al. (2009) Dev. Biol., 336:30-41; Gerhart et al. (2000) J. Cell Biol., 149:825-834; Gerhart et al. (2007) J. Cell Biol., 178:649-660). Elimination of Myo/Nog cells in the early embryo deregulates BMP signaling, produces severe malformations of the central nervous system, eyes and body wall, and disrupts myogenesis (Gerhart et al. (2011) Dev. Biol., 359:12-25; Gerhart et al. (2006) J. Cell Biol., 175:283-292; Gerhart et al. (2009) Dev. Biol., 336:30-41). Chick embryo Myo/Nog cells also respond to cells undergoing apoptosis in the epiblast and wounding of the lens epithelium by rapidly expanding and migrating to the site of injury (Gerhart et al. (2011) Dev. Biol., 359:12-25; Walker et al. (2010) Proc. Natl. Acad. Sci., 107:13730-13735).

Myo/Nog cells express MyoD mRNA and the bone morphogenetic protein inhibitor noggin. In the chick embryo, Myo/Nog cells are required for normal morphogenesis and differentiation and they respond to wounding. Herein, it was determined whether Myo/Nog cells are present in normal mammalian skin and whether they react to epidermal abrasion and tumorigenesis. Myo/Nog cells are the source of noggin in hair follicles. Myo/Nog cells are scarce within the interfollicular dermis and are absent in the epidermis of murine and human skin. Within 24 hours of abrading murine skin, Myo/Nog cells increase in number in the follicles and appear in the dermis surrounding the hair bulb and the wound. Myo/Nog cells are also recruited to the stroma of tumors formed from Ras transformed keratinocytes (Ker/Ras). Noggin blocks the inhibitory effect of BMP-4 on Ker/Ras proliferation *in vitro.* Human squamous cell carcinomas and malignant melanomas contain significantly more Myo/Nog cells than basal cell carcinomas. In human and Ker/Ras tumors, Myo/Nog cells are distinct from macrophages, granulocytes and cells expressing alpha smooth muscle actin. Myo/Nog cells are key regulators of skin homeostasis and wound healing and are diagnostic and therapeutic targets in skin cancer.

Myo/Nog cells are indispensable regulators of BMP signaling during chick embryo morphogenesis and differentiation (Gerhart et al. (2011) Dev. Biol., 359:12-25; Gerhart et al. (2006) J. Cell Biol., 175:283-292; Gerhart et al. (2009) Dev. Biol., 336:30-41). Herein, the presence of Myo/Nog cells in skin of adult mice and humans was determined. Lineage analyses will reveal whether Myo/Nog cells in the adult mouse are direct descendants of those that emerge in the epiblast of the embryo. Regardless of their origin in adult tissues, Myo/Nog cells are the primary source of noggin in the hair follicle. Noggin-modulation of BMP signaling is required for hair follicle growth and maintenance (Botchkarev et al. (2001) FASEB J., 15:2205-2214; Jamora et al. (2003) Nature 422:317-322), and therefore, Myo/Nog cells are key regulators of follicle homeostasis.

In uninjured murine and human skin, Myo/Nog cells are present in low numbers in the hair follicles and scarce within the dermis. Myo/Nog cells differ from multipotent dermal papilla cells that do not express myogenic markers *in vivo* but are induced to do so *in vitro* (Rufaut et al. (2006) J. Cell Physiol., 209:959-966; Ben-Yair et al. (2005) Development 132:689-701;
Lang et al. (2005) Nature 433:884-887; Rendl et al. (2005) PLoS Biol., 3:e331). However, they may be akin to the subpopulation of skin fibroblasts that engraft into regenerating muscle (Montanaro et al. (2003) Proc. Natl. Acad. Sci., 100:9336-9341; Pye et al. (2001) J. Anat., 198:163-173). Within 24 hours of epidermal abrasion, Myo/Nog cells increase in the hair bulb, populate the dermis surrounding the follicle and appear at the wounded surface. These behaviors of Myo/Nog cells in abraded skin are reminiscent of their response to apoptosis in the epiblast and wounding of the lens of the chick embryo (Gerhart et al. (2011) Dev. Biol., 359:12-25; Walker et al. (2010) Proc. Natl. Acad. Sci., 107:13730-13735). Without being bound by theory, mechanical tension on the follicles during the abrasion procedure, as well as cytokines and chemokines released within the injured tissue, may activate and attract Myo/Nog cells.

Both physical and inflammatory stimuli are known activators and recruiters of myofibroblasts that contract to promote wound closure (Gabbiani, G. (2003) J. Pathol., 200:500-503). Myofibroblasts also modulate the behavior of tumor cells (De Wever et al. (2008) Int. J. Cancer 123:2229-2238). Both Myo/Nog cells and myofibroblasts are capable of synthesizing MyoD, vimentin, α-SMA and myosin (Walker et al. (2010) Proc. Natl. Acad. Sci., 107:13730-13735; Gerhart et al. (2004) J. Cell Biol., 164:739-746; Gerhart et al. (2001) J. Cell Biol., 155:381-392; Strony et al. (2005) Gene Expr. Patterns 5:387-395; Gerhart et al. (2007) J. Cell Biol., 178:649-660). Multiple sources have been proposed for the origin of myofibroblasts in wounded and tumor bearing skin, including the bone marrow, blood and hair follicles (Barth et al. (2007) Curr. Stem Cell Res. Ther., 2:221-227; Direkze et al. (2006) Hematol. Oncol., 24:189-195; Gharzi et al. (2003) Exp. Dermatol., 12:126-136). Although G8 and α-SMA were detected in separate populations of cells in mammalian skin, the G8 antigen is downregulated as α-SMA expression is initiated in cultures of embryonic lens tissue (Walker et al. (2010) Proc. Natl. Acad. Sci., 107:13730-13735). Therefore, Myo/Nog cells are a source of at least some α-SMA+ myofibroblasts cells in wounded and tumor bearing skin. Myo/Nog cells are likely among the earliest responders to tissue injury and tumorigenesis given their capacity for rapid activation and migration in the epiblast, lens and abraded skin (Gerhart et al. (2011) Dev. Biol., 359:12-25; Walker et al. (2010) Proc. Natl. Acad. Sci., 107:13730-13735).

Myo/Nog cells were more abundant in sections of malignant melanomas and squamous cell carcinomas than basal cell carcinomas, indicating that the extent to which Myo/Nog cells are recruited to tumors correlates with metastatic potential. In melanoma tumors, a few Myo/Nog cells contained melanin within the cytoplasm. Myo/Nog cells may have been induced to synthesize melanin, phagocytosed apoptotic melanoma cells or engulfed melanosomes. Alternatively, Myo/Nog cells, which are capable of fusing to form multinucleated myofibers *in vitro* (Gerhart et al. (2001) J. Cell Biol., 155:381-392), may form heterokaryons with melanoma cells in a phenomenon akin to the cannibalism of lymphocytes by melanoma cells (Lugini et al. (2006) Cancer Res., 66:3629-3638).

Myo/Nog cells are likely to have multiple functions in wounds and tumors. They are the primary source of noggin in normal, abraded and tumor bearing skin. BMPs inhibit keratinocyte proliferation, and when overexpressed in the epidermis, they delay wound healing (Kaiser et al. (1998) J. Invest. Dermatol., 111:1145-1152; Blessing et al. (1996) J. Cell Biol., 135:227-239; Drozdoff et al. (1994) Proc. Natl. Acad. Sci., 91:5528-5532). In this context, Myo/Nog cells can be important for modulating the regeneration of the epidermis. Blocking BMP signaling promotes the development of skin tumors in mice (Ming et al. (2004) Genesis 39:10-25; Qiao et al. (2006) Oncogene 25:207-217; Yang et al. (2005) Cancer Res., 65:8671-8678; Sharov et al. (2009) Am. J. Pathol., 175:1303-1314; Hsu et al. (2008) Lab Invest., 88:842-855). The ability of noggin to stimulate Ker/Ras proliferation *in vitro* is consistent with these *in vivo* results and indicates that Myo/Nog cells may facilitate the growth of skin carcinomas by releasing noggin. However, BMPs stimulate the proliferation of other types of cancer cells *in vitro* and *in vivo* (Thawani et al. (2010) Neurosurgery 66:233-246). Therefore, the effect of noggin released by Myo/Nog cells may be tissue and context dependent and ultimately reflect how tumor and Myo/Nog cells integrate their inputs from multiple signaling pathways.

In addition to their production of noggin, Myo/Nog cells are capable of synthesizing contractile proteins and expanding and migrating in response to wounding and apoptosis in the chick embryo (Gerhart et al. (2011) Dev. Biol., 359:12-25; Walker et al. (2010) Proc. Natl. Acad. Sci., 107:13730-13735; Gerhart et al. (2004) J. Cell Biol., 164:739-746; Gerhart et al. (2001) J. Cell Biol., 155:381-392; Strony et al. (2005) Gene Expr. Patterns 5:387-395; Gerhart et al. (2007) J. Cell Biol., 178:649-660). Analyses of murine and human skin revealed that the number of Myo/Nog cells increases in tissue remodeling events that accompany epidermal abrasion and tumor expansion. Therefore, Myo/Nog cells play a role in mammalian wound repair, as well as the development of neoplastic lesions. Accordingly, Myo/Nog cell are useful targets for diagnosing, prognosing and treating cancer.

As stated hereinabove, cells that express MyoD, the G8 antigen and the bone morphogenetic protein (BMP) inhibitor noggin (Myo/Nog cells) are associated with cancer cells in a variety of human tumors, including carcinomas, melanomas, and sarcomas. Proliferation of some cancer cells is repressed by BMPs, and therefore, release of noggin by Myo/Nog cells may enhance tumor growth. Myo/Nog cells have the capacity to migrate to the leading edge of wounds and direct the movement of epithelial cells, indicating that that Myo/Nog cells may facilitate the invasion of carcinoma cells. Myo/Nog cells also can develop into myofibroblast-like cells that can promote tumor progression. Because Myo/Nog cells express the immunoregulatory enzyme indoleamine 2, 3-dioxygenase (IDO), they also can suppress T cell responses and/or contribute to tumor-induced tolerance and immune escape. In addition, human rhabdomyosarcoma tumors contain a subpopulation of Myo/Nog cells, and a human rhabdomyosarcoma cell line has been determined to express MyoD, G8 and noggin *in vitro.* These findings indicate that 1) Myo/Nog cells modulate the behavior of tumor and surrounding stromal cells and 2) Myo/Nog cells are a direct source of rhabdomyosarcoma stem cells.

In accordance with the instant invention, Myo/Nog cells in human cancers are targeted as adjuvant or direct therapy for reducing tumor burden. The invention is applicable to the treatment of multiple types of cancers as adjuvant or direct chemotherapy. Administration of, for example, the G8 antibody (such as a humanized version) will lead to the destruction of Myo/Nog cells in human tumors. Reduction of Myo/Nog cells with antibody may be accomplished, for example, following binding of endogenous complement or conjugating the antibody with a toxin released in the cytoplasm upon internalization. The antibody may also be conjugated with photothermal or radio frequency activated nanoparticles. The invention also includes all other methods for targeting Myo/Nog cells or the molecules they release, such as other cell type specific antibodies, drugs and small molecule inhibitors.

Identification of Myo/Nog cells in tumor tissue is a useful prognostic test for tumor grade and potential effectiveness of targeted therapy. For example, Myo/Nog cells may be identified and quantified in biopsy tissue and resected tumors (e.g., with a G8 antibody (e.g., a mouse monoclonal antibody)). The number of Myo/Nog cells in tissue sections serves as a prognostic indicator of tumor malignancy and an indicator of whether agents that target Myo/Nog cells are an appropriate form of therapy.

In accordance with the instant invention, methods of inhibiting (e.g., reducing), preventing, and/or treating cancer are provided. In a particular embodiment, the cancer is sarcoma (e.g., Ewing's sarcoma, Wilm's tumor, osteosarcoma, leiomyosarcoma, and rhabdomyosarcoma (e.g., alveolar or embryonal rhabdomyosarcoma)), a skin cancer, glioblastoma, bladder cancer, breast cancer, kidney cancer, bone cancer, colon cancer, or pancreatic cancer. In a particular embodiment, the cancer is Wilm's tumor or a rhabdomyosarcoma. Skin cancers include, without limitation, basal cell carcinomas, squamous cell carcinomas, and melanomas. In a particular embodiment, the skin cancer is a squamous cell carcinoma or a malignant melanoma. The methods may comprise reducing and/or eliminating the Myo/Nog cells in the target tissue or tumor. In certain embodiments, the method comprises administering to a subject in need thereof a therapeutically effective amount of at least one Myo/Nog targeting agent (e.g., antibody) of the instant invention. In a particular embodiment, the Myo/Nog targeting agent is conjugated to at least one toxin. The method may further comprise the administration of at least one other cancer therapy (simultaneously and/or sequentially (before and/or after)) such as radiation therapy, surgery to remove the tumor, and/or the administration of at least one other chemotherapeutic agent. In a particular embodiment, the method may further comprise administering Myo/Nog cells to the patient following treatment (e.g., Myo/Nog cells harvested from normal tissue, particularly autologous Myo/Nog cells).

Ablation of Myo/Nog cells can be achieved by various approaches. As stated hereinabove, Myo/Nog cells are progenitor cells which express MyoD, noggin, and G8 and which can give rise to skeletal muscle, develop into myofibroblasts-like cells, and/or remain as Myo/Nog cells that release Noggin. In a particular embodiment, the methods of the instant invention take advantage of the fact that Myo/Nog cells possess different molecules (e.g., at the plasma membrane) than other cells. In a particular embodiment of the instant invention, the methods of inhibiting, preventing, and/or treating cancer comprise the administration of at least one molecule comprising at least one Myo/Nog cells targeting moiety optionally with (e.g., conjugated to) at least one cytotoxic molecule). The molecule(s) of the instant invention may be contained in at least one pharmaceutically acceptable carrier. In a particular embodiment, the targeting moiety is covalently linked to the cytotoxic molecule, optionally through a linking domain. In another embodiment, the cytotoxic molecule is attached to a molecule having specific affinity for the targeting moiety (e.g., an antibody which recognizes the G8 antibody). In yet another embodiment, the targeting moiety and cytotoxic molecule are not covalently linked. For example, the method of the instant invention may comprise the administration of the G8 antibody (a Myo/Nog cell targeting molecule) and complement in a pharmaceutical composition or separate pharmaceutical compositions administered sequentially or concurrently.

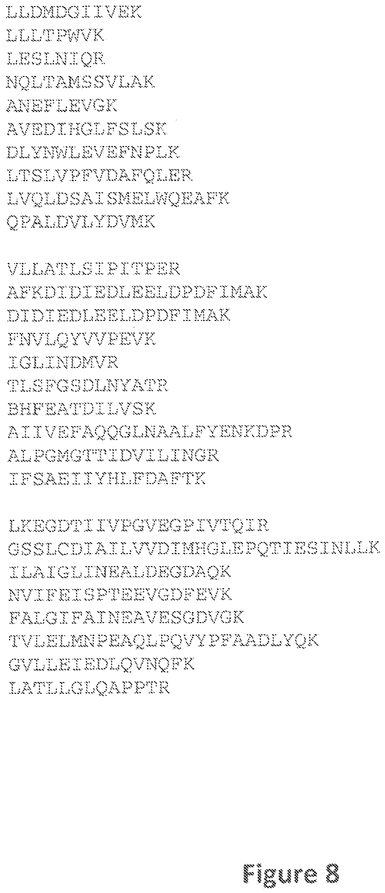

Specific targeting of Myo/Nog cells can be achieved by specifically binding cell surface molecules, e.g., with ligands and/or antibodies, which are unique to Myo/Nog cells compared to other cells (e.g., those surrounding the tumor). Cell surface targets for Myo/Nog cells include, without limitation, G8 antigen, syndecans (syndecans 1-4 (e.g., syndecan 1); e.g., GenelD Nos: 6382, 6383, 9672, and 6385 and GenBank Accession Nos. NP_001006947, NP_002989, NP_055469, and NP_002990), or heparan sulfate proteoglycan (Bayne et al. (1984) J. Cell Biol., 99:1486-1501). In a particular embodiment, the cell surface target is G8 antigen (Gerhart et al. (2001) J. Cell Biol., 155:381-392; Gerhart et al. (2004) J. Cell Biol., 164:739-746; Strony et al. (2005) Gene Expr. Patterns, 5:387-395). In a particular embodiment, the cell surface target is IQ motif containing GTPase activating protein 1 (IQGAP1; e.g., Gene ID: 8826; GenBank Accession Nos.: P46940, Q6P1N4, Q05DN7, Q5FWG8, Q96PA3, AK304337, and AK298000; UniProtKB/TrEMBL Nos. F5H7A1, A4QPB0, B4E2M0, and B4DNP4). In a particular embodiment, the cell surface target is eukaryotic translation initiation factor 3, subunit A (Eif3A; e.g., Gene ID: 8661; GenBank Accession Nos.: NP_003741.1 and NM_003750.2; UniProtKB/Swiss-Prot Nos. Q14152; Nakai et al. (2005) Exp. Cell Res., 309:137-148). In a particular embodiment, the cell surface target is eukaryotic translation initiation factor 5B (Eif5B; e.g., Gene ID: 9669; GenBank Accession Nos.: NM_015904.3 and NP_056988.3; UniProtKB/TrEMBL Nos. Q8N5A0). In a particular embodiment, the surface target is a variant (e.g., alternative splice variant) or subunit of the above identified proteins. In yet another embodiment, the targeting moiety is an antibody which is immunologically specific for an amino acid sequence provided in Figure 8. Accordingly, the targeting moiety of the instant invention specifically binds a cell surface target of Myo/Nog cells, particularly to the general exclusion of other cells (e.g., epithelial cells), particularly those cells around the tumor/cancer cells to be treated.

Myo/Nog cells can be eliminated/reduced by coupling the targeting moiety to reagents that induce cell death (e.g., a cytotoxic molecule). Cytotoxic molecules include, without limitation, complement (e.g., mouse, rat, rabbit, guinea pig, cow, horse, and human complement; blood/serum fractions containing complement; complement component(s)/protein(s); activators of complement; and the like), nanoparticles and nanotubes (e.g., photothermal or radio frequency activated nanoparticles; heat sensitive carbon nanocrystals; see e.g., Chakravarty et al. (2008) PNAS 105:8697-8702 and Cho et al. (2008) Clin. Cancer Res., 14:1310-1316), cytoxic antibiotics (e.g., calicheamicin), cationic amphipathic lytic peptides (e.g., KLA (amino acid sequence: KLAKLAKKLAKLAK (SEQ ID NO: 4)) and PTP (prostate-specific membrane antigen-targeting peptide: CQKHHNYLC (SEQ ID NO: 5))), radionuclides, chemotherapeutic agents, and toxins. Toxins can be derived from various sources, such as plants, bacteria, animals, and humans or be synthetic toxins (drugs), and include, without limitation, saprin, ricin (e.g., ricin A), abrin, ethidium bromide, diptheria toxin, Pseudomonas exotoxin, PE40, PE38, saporin, gelonin, RNAse, peptide nucleic acids (PNAs), ribosome inactivating protein (RIP) type-1 or type-2, pokeweed anti-viral protein (PAP), bryodin, momordin, chemotherapeutic agents, and bouganin. Radionuclides of the instant invention include, without limitation, positron-emitting isotopes and alpha-, beta-, gamma-, Auger- and low energy electron-emitters. In a particular embodiment, the radionuclides are alpha-emitters or auger-emitters. The radioisotopes include, without limitation: ¹³N, ¹⁸F , ³²P, ⁶⁴Cu, ⁶⁶Ga, ⁶⁷Ga, ⁶⁸Ga, ⁶⁷Cu, ⁷⁷Br, ^{80m}Br, ⁸²Rb, ⁸⁶Y, ⁹⁰Y, ⁹⁵Ru, ⁹⁷Ru, ^{99m}Tc, ¹⁰³Ru, ¹⁰⁵Ru, ¹¹¹In, ^{113m}In, ¹¹³Sn, ^{121m}Te, ^{122m}Te, ^{125m}Te, ¹²³I, ¹²⁴I, ¹²⁵I, ¹²⁶I, ¹³¹I, ¹³³I, ¹⁶⁵Tm, ¹⁶⁷Tm, ¹⁶⁸Tm, ¹⁷⁷Lu, ¹⁸⁶Re, ¹⁸⁸Re, ^{195m}Hg, ²¹¹At, ²¹²Bi, ²¹³Bi, and ²²⁵Ac. In yet another embodiment, the radionuclide containing molecule can be administered with a radiosensitizer. In a particular embodiment, the targeting moiety is conjugated to a radiosentizer.

The present invention encompasses compositions comprising 1) at least one targeting moiety optionally with (e.g., conjugated to) at least one cytotoxin and 2) at least one pharmaceutically acceptable carrier. Such compositions may be administered, in a therapeutically effective amount, to a patient in need thereof for the treatment of cancer. The compositions may further comprise one other chemotherapeutic agent. Alternatively, the other chemotherapeutic agent may be contained within a separate composition with at least one pharmaceutically acceptable carrier for sequential and/or simultaneous administration. Composition(s) of the instant invention may be contained within a kit.

Chemotherapeutic agents are compounds that exhibit anticancer activity and/or are detrimental to a cell (e.g., a toxin). Suitable chemotherapeutic agents include, but are not limited to: toxins (e.g., saporin, ricin, abrin, ethidium bromide, diptheria toxin, Pseudomonas exotoxin, and others listed above; thereby generating an immunotoxin when conjugated or fused to an antibody); alkylating agents (e.g., nitrogen mustards such as chlorambucil, cyclophosphamide, isofamide, mechlorethamine, melphalan, and uracil mustard; aziridines such as thiotepa; methanesulphonate esters such as busulfan; nitroso ureas such as carmustine, lomustine, and streptozocin; platinum complexes such as cisplatin and carboplatin; bioreductive alkylators such as mitomycin, procarbazine, dacarbazine and altretamine); DNA strand-breakage agents (e.g., bleomycin); topoisomerase II inhibitors (e.g., amsacrine, dactinomycin, daunorubicin, idarubicin, mitoxantrone, doxorubicin, etoposide, and teniposide); DNA minor groove binding agents (e.g., plicamydin); antimetabolites (e.g., folate antagonists such as methotrexate and trimetrexate; pyrimidine antagonists such as fluorouracil, fluorodeoxyuridine, CB3717, azacitidine, cytarabine, and floxuridine; purine antagonists such as mercaptopurine, 6-thioguanine, fludarabine, pentostatin; asparginase; and ribonucleotide reductase inhibitors such as hydroxyurea); tubulin interactive agents (e.g., vincristine, vinblastine, and paclitaxel (Taxol)); hormonal agents (e.g., estrogens; conjugated estrogens; ethinyl estradiol; diethylstilbesterol; chlortrianisen; idenestrol; progestins such as hydroxyprogesterone caproate, medroxyprogesterone, and megestrol; and androgens such as testosterone, testosterone propionate, fluoxymesterone, and methyltestosterone); adrenal corticosteroids (e.g., prednisone, dexamethasone, methylprednisolone, and prednisolone); leutinizing hormone releasing agents or gonadotropin-releasing hormone antagonists (e.g., leuprolide acetate and goserelin acetate); and antihormonal antigens (e.g., tamoxifen, antiandrogen agents such as flutamide; and antiadrenal agents such as mitotane and aminoglutethimide).

The compositions of the present invention can be administered by any suitable route, for example, by injection (e.g., for local (direct, including to or within a tumor) or systemic administration), oral, pulmonary, topical, nasal or other modes of administration. The composition may be administered by any suitable means, including parenteral, intramuscular, intravenous, intraarterial, intraperitoneal, subcutaneous, topical, inhalatory, transdermal, intrapulmonary, intraareterial, intrarectal, intramuscular, and intranasal administration. In a particular embodiment, the composition is administered directly to the skin. In general, the pharmaceutically acceptable carrier of the composition is selected from the group of diluents, preservatives, solubilizers, emulsifiers, adjuvants and/or carriers. The compositions can include diluents of various buffer content (e.g., Tris HCl, acetate, phosphate), pH and ionic strength; and additives such as detergents and solubilizing agents (e.g., Tween 80, Polysorbate 80), anti oxidants (e.g., ascorbic acid, sodium metabisulfite), preservatives (e.g., Thimersol, benzyl alcohol) and bulking substances (e.g., lactose, mannitol). The compositions can also be incorporated into particulate preparations of polymeric compounds such as polyesters, polyamino acids, hydrogels, polylactide/glycolide copolymers, ethylenevinylacetate copolymers, polylactic acid, polyglycolic acid, etc., or into liposomes. Such compositions may influence the physical state, stability, rate of in vivo release, and rate of in vivo clearance of components of a pharmaceutical composition of the present invention. See, e.g., Remington: The Science and Practice of Pharmacy, 21st edition, Philadelphia, PA. Lippincott Williams & Wilkins. 2005. The pharmaceutical composition of the present invention can be prepared, for example, in liquid form, or can be in dried powder form (e.g., lyophilized for later reconstitution).

As used herein, "pharmaceutically acceptable carrier" includes any and all solvents, dispersion media and the like which may be appropriate for the desired route of administration of the pharmaceutical preparation, as exemplified in the preceding paragraph. The use of such media for pharmaceutically active substances is known in the art. Except insofar as any conventional media or agent is incompatible with the molecules to be administered, its use in the pharmaceutical preparation is contemplated.

The dose and dosage regimen of the molecule of the invention that is suitable for administration to a particular patient may be determined by a physician considering the patient's age, sex, weight, general medical condition, and the specific condition and severity thereof for which the antibody is being administered. The physician may also consider the route of administration, the pharmaceutical carrier, and the molecule's biological activity.

Selection of a suitable pharmaceutical preparation depends upon the method of administration chosen. For example, the molecules of the invention may be administered by direct injection into any cancerous tissue or into the area surrounding the cancer. In this instance, a pharmaceutical preparation comprises the molecules dispersed in a medium that is compatible with the cancerous tissue.

Molecules of the instant invention may also be administered parenterally by intravenous injection into the blood stream, or by subcutaneous, intramuscular, intrathecal, or intraperitoneal injection. Pharmaceutical preparations for parenteral injection are known in the art. If parenteral injection is selected as a method for administering the molecules, steps should be taken to ensure that sufficient amounts of the molecules reach their target cells to exert a biological effect. The lipophilicity of the molecules, or the pharmaceutical preparation in which they are delivered, may have to be increased so that the molecules can arrive at their target locations. Methods for increasing the lipophilicity of a molecule are known in the art. In a particular embodiment, if a small form of the antibody is to be administered, including but not limited to a Fab fragment, a Dab, an scFv or a diabody, it may be conjugated to a second molecule such as, but not limited to polyethylene glycol (PEG) or an albumin-binding antibody or peptide to prolong its retention (e.g., in blood).

Pharmaceutical compositions containing a compound of the present invention as the active ingredient in intimate admixture with a pharmaceutical carrier can be prepared according to conventional pharmaceutical compounding techniques. The carrier may take a wide variety of forms depending on the form of preparation desired for administration, e.g., intravenous, oral, topical, or parenteral. In preparing the molecule in oral dosage form, any of the usual pharmaceutical media may be employed, such as, for example, water, glycols, oils, alcohols, flavoring agents, preservatives, coloring agents and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form in which case solid pharmaceutical carriers are obviously employed. If desired, tablets may be sugar-coated or enteric-coated by standard techniques. For parenterals, the carrier will usually comprise sterile water, though other ingredients, for example, to aid solubility or for preservative purposes, may be included. Injectable suspensions may also be prepared, in which case appropriate liquid carriers, suspending agents and the like may be employed.

A pharmaceutical preparation of the invention may be formulated in dosage unit form for ease of administration and uniformity of dosage. Dosage unit form, as used herein, refers to a physically discrete unit of the pharmaceutical preparation appropriate for the patient undergoing treatment. Each dosage should contain a quantity of active ingredient calculated to produce the desired effect in association with the selected pharmaceutical carrier. Procedures for determining the appropriate dosage unit are well known to those skilled in the art. Dosage units may be proportionately increased or decreased based on the weight of the patient. Appropriate concentrations for alleviation of a particular pathological condition may be determined by dosage concentration curve calculations, as known in the art. The appropriate dosage unit for the administration of the molecules of the instant invention may be determined by evaluating the toxicity of the molecules in animal models. Various concentrations of pharmaceutical preparations may be administered to mice with transplanted human tumors, and the minimal and maximal dosages may be determined based on the results of significant reduction of tumor size and side effects as a result of the treatment. Appropriate dosage unit may also be determined by assessing the efficacy of the treatment in combination with other standard chemotherapies. The dosage units of the molecules may be determined individually or in combination with each chemotherapy according to greater shrinkage and/or reduced growth rate of tumors.

The pharmaceutical preparation comprising the molecules of the instant invention may be administered at appropriate intervals, for example, at least twice a day or more until the pathological symptoms are reduced or alleviated, after which the dosage may be reduced to a maintenance level. The appropriate interval in a particular case would normally depend on the condition of the patient.

According to another aspect of the instant invention, methods for detecting, imaging, diagnosing, and/or determining an increased risk for cancer in a patient are provided. In a particular embodiment, the methods comprise administering to a patient a targeting moiety of the instant invention (e.g., an agent which specifically targets Myo/Nog cells) conjugated to at least one detectable label. In a particular embodiment, the methods comprise detecting the presence of Myo/Nog cells (e.g., in, among and/or around tumor cells) in a biological sample with the molecules of the instant invention. In a particular embodiment, the method comprises incubating a biological sample with at least one antibody of the instant invention, optionally comprising at least one detectable label.

Detectable labels include, for example, chemiluminescent moieties, bioluminescent moieties, fluorescent moieties, contrast agents, radionuclides, isotopes (e.g., radioisotopes (e.g., ³H (tritium) and ¹⁴C) or stable isotopes (e.g., ²H (deuterium), ¹¹C, ¹³C, ¹⁷O and ¹⁸O), optical agents for imaging, and metals (e.g., gold). Examples of detectable labels include, without limitation, paramagnetic or superparamagnetic ions for detection by MRI imaging. Paramagnetic ions include, without limitation, Gd(III), Eu(III), Dy(III), Pr(III), Pa(IV), Mn(II), Cr(III), Co(III), Fe(III), Cu(II), Ni(II), Ti(III), and V(IV). Fluorescent agents include, without limitation, fluorescein and rhodamine and their derivatives. Optical agents include, without limitation, derivatives of phorphyrins, anthraquinones, anthrapyrazoles, perylenequinones, xanthenes, cyanines, acridines, phenoxazines and phenothiazines.

As stated above, the molecules of the invention may be used to 1) diagnose (e.g., determine an increased risk of) cancer in patient, 2) determine the prognosis of a patient, including A) stage and grade of a tumor (particularly whether the cancer is metastatic or likely to be metastatic) and/or B) its potential sensitivity to Myo/Nog therapy of the instant invention, 3) determine the origin of a tumor, 4) determine the efficacy of a treatment of a patient. In certain embodiments, the molecules are utilized to detect the presence of Myo/Nog cells in a biological sample from a patient. The biological sample may include biopsies of various tissues including, without limitation: skin, muscle, breast, prostate, cervical, ovarian, testicular, and pulmonary. Cellular examples of biological samples include tumor cells, skin cells, muscle cells, blood cells, ovarian cells, prostate cells, breast cells, testicular cells, cervical cells, and lung cells. The biological sample may also be a biological fluid. Many immunological assays are well known in the art for assaying of biological samples for the presence of a certain protein (e.g., Myo/Nog specific surface protein) including, without limitation: immunoprecipitations, radioimmunoassays, enzyme-linked immunosorbent assays (ELISA), immunohistochemical assays, Western blot and the like.

The presence of Myo/Nog cells in a biological sample is indicative of the presence of cancer and indicative of metastases, particularly when present in quantities greater than that of normal healthy subjects. The loss of Myo/Nog cells in a patient, particularly one undergoing treatment, over time is indicative of remission (i.e., successful treatment), while the lack of change in Myo/Nog cell levels in a patient undergoing treatment is indicative of resistance to the therapy and indicates that a different therapeutic strategy could be employed. Similarly, the gain of Myo/Nog cells in a patient over time can be indicative of recurrence. Additionally, the imaging techniques described hereinabove may be employed to monitor the size of the tumor to determine the efficacy of a treatment. In a particular embodiment of the invention, other cancer diagnostic assays can be performed to confirm the results obtained with the instant invention.

In accordance with another aspect of the instant invention, a biological sample (e.g., a tumor sample) may be obtained from a subject and the presence of Myo/Nog cells determined. The number of Myo/Nog cells may be correlated with tumor grade. In a particular embodiment, the number of Myo/Nog cells in the biological sample is compared to the number of Myo/Nog cells in a corresponding biological sample from a healthy individual to determine the modulation of Myo/Nog cells in the tumor. Subjects comprising the tumor may be treated with agents to modulate the activity of Myo/Nog cells to normal, healthy levels.

### Definitions

The following definitions are provided to facilitate an understanding of the present invention:
The singular forms "a," "an," and "the" include plural referents unless the context clearly dictates otherwise.

An "antibody" or "antibody molecule" is any immunoglobulin, including antibodies and fragments thereof, that binds to a specific antigen. The term includes polyclonal, monoclonal, chimeric, single domain (Dab) and bispecific antibodies. As used herein, antibody or antibody molecule contemplates recombinantly generated intact immunoglobulin molecules and immunologically active portions of an immunoglobulin molecule such as, without limitation: Fab, Fab', F(ab')₂, F(v), scFv, scFv₂, scFv-Fc, minibody, diabody, tetrabody, single variable domain (e.g., variable heavy domain, variable light domain), and bispecific. Dabs can be composed of a single variable light or heavy chain domain. The instant invention also encompasses Affibody® molecules (Affibody, Bromma, Sweden) and peptabodies (Terskikh et al. (1997) PNAS 94:1663-1668). In a certain embodiment of the invention, the variable light domain and/or variable heavy domain specific for target molecule on Myo/Nog cells are inserted into the backbone of the above mentioned antibody constructs. Methods for recombinantly producing antibodies are well-known in the art. "Fv" is an antibody fragment which contains an antigen-recognition and -binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although often at a lower affinity than the entire binding site. "Single-chain Fv" or "scFv" antibody fragments comprise the V_{H} and V_{L} domains of an antibody, wherein these domains are present in a single polypeptide chain. Generally, the Fv polypeptide further comprises a polypeptide linker between the V_{H} and V_{L} domains which enables the scFv to form the desired structure for antigen binding. The term "diabodies" refers to small antibody fragments with two antigen-binding sites, which fragments comprise a heavy-chain variable domain (V_{H}) connected to a light-chain variable domain (V_{L}) on the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Holliger et al., (1993) Proc. Natl. Acad. Sci. USA, 90: 6444-6448. In yet another embodiment, the antibody is humanized.

With respect to antibodies, the term "immunologically specific" refers to antibodies that bind to one or more epitopes of a protein or compound of interest, but which do not substantially recognize and bind other molecules in a sample containing a mixed population of antigenic biological molecules. The term "specifically binds" refers to the binding of a polypeptide or compound of interest to a target polypeptide or compound while not substantially recognizing and binding other molecules in a sample containing a mixed population of biological molecules. For example, a "specific binding pair" comprises a specific binding member and a binding partner which have a particular specificity for each other and which in normal conditions bind to each other in preference to other molecules.

The term "conjugated" refers to the joining by covalent or noncovalent means of two molecules or compounds of the invention. The molecules may be joined by a linker domain.

The term "linker domain" refers to a chemical moiety comprising a covalent bond or a chain of atoms that covalently attaches the targeting moiety to the cytotoxin. In a particular embodiment, the linker may contain from 0 (i.e., a bond) to about 500 atoms, about 1 to about 100 atoms, or about 1 to about 50 atoms. Exemplary linkers may comprise at least one optionally substituted; saturated or unsaturated; linear, branched or cyclic alkyl, alkenyl, or aryl group. The linker may also be a polypeptide (e.g., from about 1 to about 20 amino acids).

The term "radiosensitizer", as used herein, is defined as a molecule administered to animals in therapeutically effective amounts to increase the sensitivity of the cells to radiation. Radiosensitizers are known to increase the sensitivity of cells to the toxic effects of radiation. Radiosensitizers include, without limitation, 2-nitroimidazole compounds, and benzotriazine dioxide compounds, halogenated pyrimidines, metronidazole, misonidazole, desmethylmisonidazole, pimonidazole, etanidazole, nimorazole, mitomycin C, RSU 1069, SR 4233, EO9, RB 6145, nicotinamide, 5-bromodeoxyuridine (BUdR), 5-iododeoxyuridine (IUdR), bromodeoxycytidine, fluorodeoxyuridine (FudR), hydroxyurea, cisplatin, and therapeutically effective analogs and derivatives of the same.

"Pharmaceutically acceptable" indicates approval by a regulatory agency of the Federal or a state government or listed in the U.S. Pharmacopeia or other generally recognized pharmacopeia for use in animals, and more particularly in humans.

A "carrier" refers to, for example, a diluent, adjuvant, preservative (e.g., Thimersol, benzyl alcohol), anti-oxidant (e.g., ascorbic acid, sodium metabisulfite), solubilizer (e.g., Tween 80, Polysorbate 80), emulsifier, buffer (e.g., Tris HCl, acetate, phosphate), antimicrobial, bulking substance (e.g., lactose, mannitol), excipient, auxilliary agent or vehicle with which an active agent of the present invention is administered. Pharmaceutically acceptable carriers can be sterile liquids, such as water and oils, including those of petroleum, animal, vegetable or synthetic origin. Water or aqueous saline solutions and aqueous dextrose and glycerol solutions are preferably employed as carriers, particularly for injectable solutions. Suitable pharmaceutical carriers are described in Remington: The Science and Practice of Pharmacy, 21st Edition, (Lippincott, Williams and Wilkins; 2005); Liberman, et al., Eds., Pharmaceutical Dosage Forms, Marcel Decker, New York, N.Y., 1980; and Rowe, et al., Eds., Handbook of Pharmaceutical Excipients (6th Ed.), Pharmaceutical Pr, 2009.

As used herein, the term "prevent" refers to the prophylactic treatment of a subject who is at risk of developing a condition (e.g., cancer) resulting in a decrease in the probability that the subject will develop the condition.

As used herein, a "biological sample" refers to a sample of biological material obtained from a subject, preferably a human subject, including a tissue, a tissue sample, a cell sample, a tumor sample, and a biological fluid (e.g., blood, urine, or amniotic fluid).

As used herein, "diagnose" refers to detecting and identifying a disease or disorder in a subject. The term may also encompass assessing or evaluating the disease or disorder status (progression, regression, stabilization, response to treatment, etc.) in a patient known to have the disease or disorder.

As used herein, the term "prognosis" refers to providing information regarding the impact of the presence of a disease or disorder (e.g., as determined by the diagnostic methods of the present invention) on a subject's future health (e.g., expected morbidity or mortality, the likelihood of getting diabetes, and the risk of cardiovascular disease). In other words, the term "prognosis" refers to providing a prediction of the probable course and outcome of a disease/disorder or the likelihood of recovery from the disease/disorder.

The term "treat" as used herein refers to any type of treatment that imparts a benefit to a patient afflicted with a disease or disorder, including improvement in the condition of the patient (e.g., in one or more symptoms), delay in the progression of the condition, etc.

The following examples provide illustrative methods of practicing the instant invention, and are not intended to limit the scope of the invention in any way.

### EXAMPLE 1

### METHODS

### Skin Dissection and Abrasion

Skin was excised from the dorsum of nine FVB seven week-old mice whose follicles were in telogen phase. Anagen phase skin was excised from 14 week old mice. Regenerative epidermal growth was induced by abrasion of the dorsal skin of six FVB mice (Hayes et al. (2011) Carcinogenesis 32:1340-1348). Briefly, a two cm² area of the shaved and depilated dry dorsum was gently abraded with a felt wheel on a motor tool, leaving the exposed dermis shiny, pink and bloodless. The abraded and adjacent nonabraded dorsal skin was excised 24 hours later. Tissue was fixed in 4% paraformaldehyde, embedded in paraffin and sectioned at 5 µm.

### Subcutaneous Injection of v-Ras Infected Keratinocytes (Ker/Ras Cells) into Syngeneic Mice

Primary cultures of epidermal keratinocytes were prepared from 3-4 day old FVB mouse skin by a trypsin flotation method (Hennings et al. (1980) Cell 19:245-254; Yuspa et al. (1974) Exp. Cell Res., 86:95-105). Cells were plated at 3.5 x 10⁶ cells per 60 mm dish in low calcium Eagle's Minimum Essential Medium (EMEM) (BioWhittaker, Walkersville, MD) supplemented with 8% chelex-treated fetal bovine serum and 0.05 mM calcium, and grown at 35°C with 5% CO₂. Keratinocytes were retrovirally infected with v-Ras and cultured for 3 days (Hayes et al. (2006) Oncogene 25:1543-1553; Clifford et al. (1995) Cancer Res., 55:1680-1686). Ker/Ras cells were subcutaneously (s.c.) injected beneath the dorsal skin of FVB mice (4.0 x 10⁶ cells per injection) and observed daily for tumor development until sacrificed five weeks later. Tumors and adjacent skin were excised from 11 mice and embedded in paraffin.

### In Vitro Proliferation Assays

Primary cultures of keratinocytes were infected with a v-Ha-ras retrovirus and cultured for 48 hours in low calcium EMEM containing 1% fetal bovine serum with or without 100 ηg/ml human recombinant BMP-4 and/or 150 ηg/ml noggin (PeproTech, Rocky Hill, NJ) for 48 hours. Cultures were labeled for 60 minutes with [³H]thymidine (Amersham; 2µCi/ml; 1 µM final concentration) and chased with 0.1% thymidine in PBS. Radiolabel incorporation into the acid-insoluble fraction was determined by liquid scintillation counting of the final perchloric acid extract. The amount of DNA was determined by the diphenylamine reaction. Cell proliferation was assessed by the amount of [³H]thymidine incorporated (cpm) normalized to milligrams of DNA.

### Human Tissue

Human tissue arrays (#SK721, 24 cases/72 cores) were obtained from US Biomax, Inc. (Rockville, MD). Each array contained three 5 µm sections from five, six and seven cases of basal cell carcinoma, squamous cell carcinoma and malignant melanoma, respectively, and three cases each of normal dermal tissue adjacent to cancer and normal dermal tissue. Non-array human skin tissue was obtained from a subject undergoing breast reduction surgery and resected squamous cell carcinoma.

### Immunofluorescence Localization and In Situ Hybridization

Sections were double labeled for the G8 antigen and mRNA by incubating with the G8 IgM mAb and goat anti-mouse IgM µ chain antibodies conjugated with DyLight® 488 (Jackson ImmunoResearch, West Grove, PA), followed by incubation in Cy3 labeled 3DNATM dendrimers (Genisphere, LLC, Hatfield, PA) conjugated with an antisense cDNA sequence to MyoD or noggin mRNA (Gerhart et al. (2006) J. Cell Biol., 175:283-292; Gerhart et al. (2009) Dev. Biol., 336:30-41; Gerhart et al. (2004) J. Cell Biol., 164:739-746). The following anti-sense sequences were conjugated to dendrimers: mouse MyoD1 (M84918: 5'-GAAACACGGATCATCATAGAAGTCGTCTGCTGTCTC-3'; SEQ ID NO: 1) (Pinney et al. (1988) Cell 53:781-93), human MyoD1 (NM_002478.4: 5'-CTGTCCGGCCTGATTTGTGGTTAAGGA-3'; SEQ ID NO: 2) and mouse noggin (NM_008711.2: 5'-TCTCGTTCAGATCCTTCTCCTTAGGGTCAAA-3'; SEQ ID NO: 3) (Tonegawa et al. (1998) Dev. Biol., 202:172-182).

Double labeling was also carried out with the G8 mAb and a goat antiserum to mouse noggin (AF719, R&D Systems, Minneapolis, MN), the F4/80 IgG mAb (AbD SeroTec, Oxford, UK), alpha smooth muscle actin (a-SMA) IgG conjugated with fluorescein (Sigma-Aldrich, St. Louis, MO), anti-acidic and -basic cytokeratins AE1/AE3 IgG mAb (Millipore, Billerica, MA), anti-CD44 conjugated with fluorescein (eBiosciences, San Diego, CA), and a kit for terminal deoxynucleotidyl transferase dUTP nick end labeling (TUNEL) (Roche Diagnostics, Mannheim, Germany). Secondary antibodies included: goat anti-mouse or anti-rat IgG-fluorescein (Jackson ImmunoResearch), goat anti-mouse IgM µ chain antibodies with DyLight® 488 or DyLight® 549 and donkey anti-goat IgG-DyLight® 488 (Jackson ImmunoResearch,). Nuclei were stained with Hoechst dye 33258 (Sigma-Aldrich). Parallel sections were incubated with secondary antibodies alone to determine the level of background fluorescence or Hematoxylin 7211 and Eosin-Y (Richard-Allan Scientific, Kalamazoo, MI). Sections were mounted in Gelmount (Biomeda, Foster City, CA) or Elvanol (Dupont, Wilmington, DE).

Analyses were carried out with a Nikon Eclipse E800 epifluorescence microscope (Optical Apparatus). Images were captured with the Evolution QE Optronics video camera (Media Cybernetics) and Image Pro Plus image analysis software program (Phase 3 Imaging Systems). Figures were annotated and adjusted for brightness and contrast with the Adobe Photoshop 6.0 software program.

### Statistical Analyses

The Wilcoxon rank sum test was used to perform pairwise comparisons of the numbers of G8+ cells in sections of normal dermal and tumor tissue in the human tissue arrays. The nonparametric Kruskal-Wallis test was applied for comparisons of the number of G8+ cells and the extent of tumor invasion (T value).

### RESULTS

### Myo/Nog cells are associated with the hair follicles in murine skin

Myo/Nog cells were identified in mice in which the skin was in resting, telogen phase by double labeling tissue sections for the G8 antigen and MyoD mRNA, noggin mRNA or noggin protein. G8+/MyoD+ and G8+/noggin+ cells were associated with the hair bulb of the follicles (Fig. 1B and C). In each section, 25-50% of the follicles contained two to four Myo/Nog cells which were present within the dermal sheath, dermal papillae and between the epithelial cells of the root sheath (Fig. 1B). Anagen phase follicles had more Myo/Nog cells than telogen follicles (Fig. 1L). Myo/Nog cells were rarely found in the follicles above the hair bulb or in the interfollicular dermis, and none were observed within the epidermis (Fig. 1D and E). Adipose tissue within the hypodermis and skeletal muscle below the hypodermis contained a few Myo/Nog cells. Less than one percent of the stained cells in double labeled sections contained G8, MyoD mRNA or noggin mRNA alone. Noggin+/G8- cells were the most common of the single labeled populations. These results demonstrate that Myo/Nog cells are the primary source of noggin in telogen phase skin where they are primarily associated with the hair bulb.

### Myo/Nog cells rapidly accumulate following epidermal abrasion

Within 24 hours of epidermal abrasion, the number of G8+/MyoD+/noggin+ cells had increased dramatically in the hair bulbs (Fig. 1G and H). Some Myo/Nog cells appeared to be migrating from the follicles into the surrounding dermis (Fig. 1H). Follicles adjacent to the abraded area displayed an increase in Myo/Nog cells in the hair bulb, whereas the number of Myo/Nog cells in the follicles further away from the abraded area was similar to that found in non-wounded mice. Clusters of Myo/Nog cells were present at the wounded surface of the dermis (Fig. 1I and J). Less than one percent of the stained cells expressed only one marker of Myo/Nog cells.

### Myo/Nog cells are present in the stroma of Ker/Ras tumors

FVB mice were subcutaneously injected with Ker/Ras cells to examine the behavior of Myo/Nog cells in response to carcinoma cells. Five weeks following injection, Ker/Ras cells had formed well-encapsulated tumors of various sizes ranging from 100 to 500 mm³. Myo/Nog cells either completely surrounded the tumor or were clustered in a portion of the tumor stroma beneath the basal layer of Ker/Ras cells (Fig. 2B-D). Few Myo/Nog cells had invaded the tumor epithelial compartment. All G8+ cells expressed MyoD and noggin and no cells were labeled for either marker alone (Fig. 2C and D). The number of Myo/Nog cells was elevated in approximately 75% of the hair follicles overlying Ker/Ras tumors (Fig. 2E and F) compared to follicles lateral to the tumors which had approximately the same number of Myo/Nog cells as non-tumor bearing skin.

Myo/Nog cells were adjacent to TUNEL+ apoptotic cells in the tumor stroma (Fig. 2B). A few G8+ cells also stained with TUNEL reagents. No G8+ cells in the tumor stroma were positive for the F4/80 antigen, a marker of the monocyte/macrophage lineage (Fig. 2G). The nuclei of Myo/Nog cells were round (Fig. 2), and therefore, G8+ cells did not appear to be granulocytes with segmented nuclei. Ker/Ras tumor sections were also double labeled for G8 and α-SMA, a marker of myofibroblasts and smooth muscle cells. Neither G8, MyoD nor noggin was detected in α-SMA+ cells that were adjacent to the basal layer of the tumor and in the walls of blood vessels (Fig. 2I).

### Noggin and BMP-4 Have Opposing Effects on Ker/Ras Proliferation In Vitro

A potential role of Myo/Nog cells in the tumor stroma was explored by testing the effects of BMP-4 and noggin on Ker/Ras cell growth in vitro (Fig. 2K). Addition of BMP-4 to the medium reduced incorporation of [³H]thymidine into DNA. Exogenous noggin blocked the inhibitory effect of BMP-4 on DNA synthesis in Ker/Ras cells but did not affect their growth in the absence of BMP-4. These data indicate that stromal Myo/Nog cells function, at least in part, to promote Ker/Ras tumor growth by blocking BMP signaling.

### Myo/Nog cells are present in normal and tumor bearing human skin

A few Myo/Nog cells were associated with the hair bulb in sections of normal human skin (Fig. 3B). Myo/Nog cells were rarely detected in the interfollicular dermis and were not found within the epidermis. Myo/Nog cells were also present in human basal cell carcinoma, squamous cell carcinoma and malignant melanoma tumor tissue (Table 1 and Fig. 3). All G8+ cells in one tissue array expressed MyoD mRNA (Fig. 3F and I). Noggin protein was detected in 73% of the G8+ cells present in three tissue arrays (Fig. 3D, H, and J). The nuclei of most Myo/Nog cells in all three types of tumors were small and round (Fig. 3). As was the case in murine Ker/Ras tumors, antibodies to the G8, F4/80, α-SMA and cytokeratins labeled separate populations of cells in human skin tumors (Fig. 3K-P).

**Table 1: Prevalence of Myo/Nog cells in arrays of normal and tumor tissue from human skin. Five tissue arrays were labeled with the G8 mAb. Values on the first row of each subject are the total number of G8+ cells found in all sections from the same subject. The mean ± standard deviation of G8+ cells per section is listed below the total number of G8+ cells. Fifteen sections were scored for each subject except #6 with squamous cell carcinoma (n = 13) and #2 with malignant melanoma (n = 12). *The total number of G8+ cells was calculated from the predicted number of G8+ cells in one section + the total number of G8+ cells in the remaining sections. Predicted number = total number of G8+ cells in 20 fields X total number of fields ÷ 20. There were 65 and 67 fields in #6 and #5, respectively.**

| Subject | Normal Dermis | Normal Dermis Adj. to Tumor | Basal Cell Carcinoma | Squamous Cell Carcinoma | Malignant Melanoma |
|---|---|---|---|---|---|
| **1** | 7 1 ± 1 | 3 0.2 ± 1 | 2 0.1 ± 0.5 | 93 6 ± 6 | 61 4 ± 10 |
| **2** | 9 1 ± 2 | 31 2 ± 6 | 27 2 ± 6 | 806 54 ± 60 | 603 20 ± 40 |
| **3** | 10 1 ± 2 | 28 2 ± 4 | 34 2 ± 9 | 169 11 ± 30 | 300 20 ± 40 |
| **4** | | | 21 1 ± 4 | 972 50 ± 22 | 137 9 ± 13 |
| **5** | | | 59 54 ± 14 | 779 52 ± 102 | 299 15 ± 19 |
| **6** | | | | *3722 286 ± 839 | *2988 199 ± 707 |
| **7** | | | | | 207 14 ± 27 |

Myo/Nog cells were found in sections of tumors from all subjects, although their numbers varied between subjects and tumor type (Table 1). Significantly, more Myo/Nog cells were found in squamous cell carcinomas and malignant melanomas than basal cell carcinomas and normal dermal tissue (p < 0.0001), and in squamous cell carcinomas than malignant melanomas (p = 0.0185). Variation in the number of Myo/Nog cells was also observed in sections from the same subject, indicating that Myo/Nog cells are not uniformly distributed in tumors. In squamous cell carcinomas, but not basal cell carcinomas or malignant melanomas, the number of G8+ cells correlated with the extent of tumor invasion (T value) (p < 0.0001).

Although the number of Myo/Nog cells in normal dermal tissue and basal cell carcinomas was not significantly different, there were differences in their distribution. Whereas Myo/Nog cells were rarely found in the normal dermis, they were present in clusters of various sizes underlying basal cell carcinoma cells (Fig. 3D). In squamous cell carcinomas, Myo/Nog cells were mostly concentrated within the stromal compartment; however, some were found among the tumor cells themselves (Fig. 3E-H). A greater degree of Myo/Nog cell infiltration among tumor cells was observed in malignant melanomas than carcinomas (Fig. 3I-K). In heavily pigmented melanoma tissue, some G8+/MyoD+ and G8+/noggin+ cells appeared to have melanin in the cytoplasm (Fig. 3J).

### EXAMPLE 2

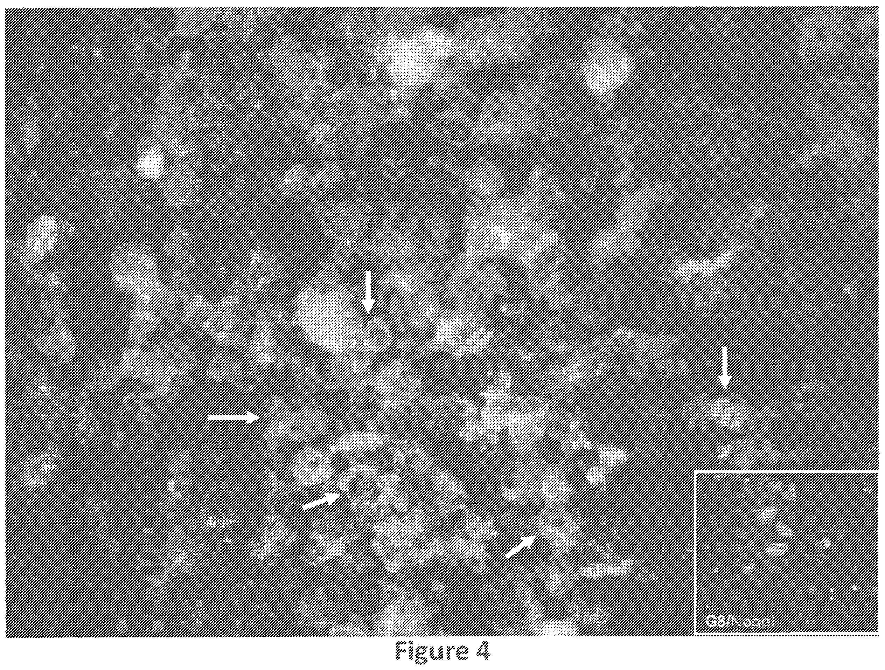

Figure 4 shows that Myo/Nog cells express the Pax3/FOXO1 translocation in a mouse model of alveolar rhabdomyosarcoma (ARMS). The Pax3:FOXO1 translocation is present in 80% of patients with ARMS. Myf6 drives this translocation in mice. Fluorescent protein is co-expressed with the translocation and sections were stained with the G8 antibody. Other sections were double labeled for G8 and noggin (inset of Figure 4). The data indicate that G8-positive cells co-express noggin and a subpopulation of ARMS cells co-expresses the G8 antigen and the Pax3:FOXO1 translocation.

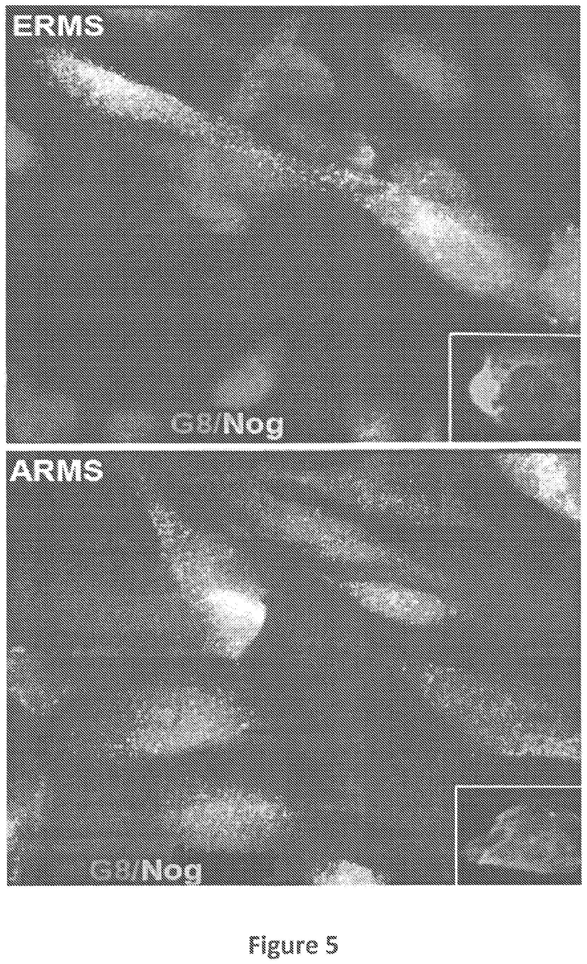

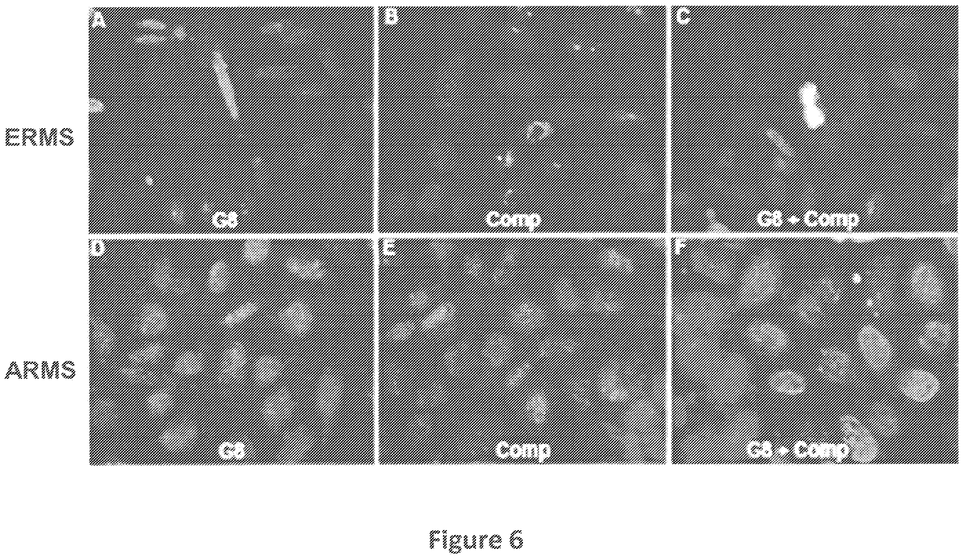

Figure 5 shows that human rhabdomyosarcoma cells express G8 and noggin. More specifically, human embryonal and alveolar rhabdomyosarcoma cells (ERMS and ARMS, respectively) were stained with antibodies to G8 and noggin. ERMS and ARMS cell cultures contained G8+/noggin+ cells. Notably, the percent of G8+ cells in ERMS was 10% ± 5 and the percent of G8+ cells in ARMS was 91% ± 8. The G8+ ERMS and ARMS cells were killed with G8 antibody and complement (Figure 6). Specifically, ERMS and ARMS cultures were incubated with the G8 antibody, complement that lyses antibody bound cells, or both. The cells were then labeled for G8 and TUNEL. The results presented in Figure 6 demonstrate that treatment with G8 and complement specifically kills cells that express the G8 antigen.

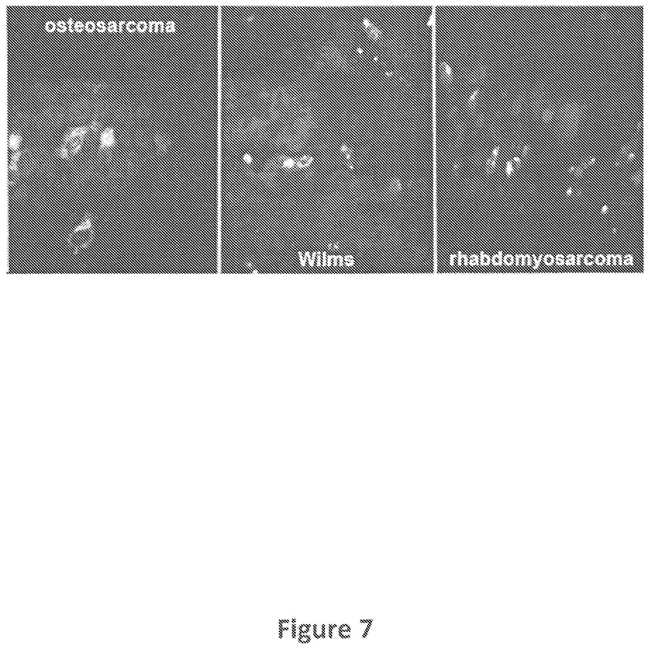

Figure 7 shows presence of G8-positive cells in various sarcomas. Sections of human osteosarcoma, Wilms and rhabdomyosarcoma tumors were labeled with antibodies to G8 and noggin. G8+/noggin+ cells were a subpopulation within the tumors. Indeed, G8 expressing cells have been identified in osteosarcoma, Wilms, rhabdomyosarcoma, leiomyocarcoma, squamous cell, melanoma, basal cell, and Ewings tumors.
The following represent possible embodiments according to the invention:
1. A method of inhibiting cancer in a subject in need thereof, said method comprising administering to the subject a therapeutically effective amount of at least one Myo/Nog cell targeting molecule and, optionally, at least one cytotoxic molecule.
2. The method of paragraph 1, wherein said at least Myo/Nog cell targeting molecule is conjugated to said at least one cytotoxic molecule.
3. The method of paragraph 1, wherein said at least one Myo/Nog cell targeting molecule and at least one cytotoxic molecule are contained within a composition further comprising at least one pharmaceutically acceptable carrier.
4. The method of paragraph 1, wherein said cancer selected from the group consisting of skin cancer, sarcoma, and rhabdomyosarcoma.
5. The method of paragraph 4, wherein said skin cancer is squamous cell carcinoma or malignant melanoma.
6. The method of paragraph 1, further comprising the administration of at least one other chemotherapeutic agent or radiation therapy to the subject.
7. The method for paragraph 1, wherein said Myo/Nog cell targeting molecule specifically binds to G8 antigen.
8. The method of paragraph 1, wherein said cytotoxic molecule is selected from group consisting of complement, photothermal or radio frequency activated nanoparticles, heat sensitive carbon nanocrystals, cytoxic antibiotics, cationic amphipathic lytic peptides, radionuclides, chemotherapeutic agents, and toxins.
9. The method of paragraph 1, wherein said composition is administered directly to a tumor or the surrounding tissue.
10. The method of paragraph 7, wherein said Myo/Nog cell targeting molecule is the G8 antibody.
11. A method of detecting an increase risk for cancer or metastatic potential in a subject, said method comprising detecting Myo/Nog cells in a biological sample obtained from a subject,
   wherein an increase in Myo/Nog cells in the biological sample compared to a corresponding biological sample in a normal subject is indicative of an increased risk of cancer or metastatic potential in said subject.
12. The method of paragraph 11, wherein said method further comprises contacting the biological sample with at least one Myo/Nog cell targeting molecule conjugated to at least one detection molecule.

While certain of the preferred embodiments of the present invention have been described and specifically exemplified above, it is not intended that the invention be limited to such embodiments. Various modifications may be made thereto without departing from the scope and spirit of the present invention, as set forth in the following claims.

## Claims

1. A Myo/Nog cell targeting molecule for use in treating cancer.

2. A Myo/Nog cell targeting molecule for use in treating cancer according to claim 1, wherein at least one Myo/Nog cell targeting molecule is for administration in combination with at least one cytotoxic molecule.

3. A Myo/Nog cell targeting molecule for use in treating cancer according to claim 2, wherein said Myo/Nog cell targeting molecule is conjugated to said at least one cytotoxic molecule.

4. A Myo/Nog cell targeting molecule for use in treating cancer according to claim 2, wherein said Myo/Nog cell targeting molecule and at least one cytotoxic molecule are contained within a composition further comprising at least one pharmaceutically acceptable carrier.

5. A Myo/Nog cell targeting molecule for use in treating cancer according to claim 1, wherein said cancer is selected from the group consisting of skin cancer, sarcoma, and rhabdomyosarcoma.

6. A Myo/Nog cell targeting molecule for use in treating cancer according to claim 5, wherein said skin cancer is squamous cell carcinoma or malignant melanoma.

7. A Myo/Nog cell targeting molecule for use in treating cancer according to claim 1, for administration in combination with a chemotherapeutic agent or radiation therapy.

8. A Myo/Nog cell targeting molecule for use in treating cancer according to claim 1, wherein said Myo/Nog cell targeting molecule is an antibody specific for the G8 antigen, a syndecan, or heparin sulfate proteoglycan.

9. A Myo/Nog cell targeting molecule for use in treating cancer according to claim 1, wherein said Myo/Nog cell targeting molecule specifically binds to G8 antigen.

10. A Myo/Nog cell targeting molecule for use in treating cancer according to claim 1, wherein said Myo/Nog cell targeting molecule is an antibody which is immunologically specific for an amino acid sequence provided in Figure 8.

11. A Myo/Nog cell targeting molecule for use in treating cancer according to claim 2, wherein said cytotoxic molecule is selected from group consisting of complement, photothermal or radio frequency activated nanoparticles, heat sensitive carbon nanocrystals, cytotoxic antibiotics, cationic amphipathic lytic peptides, radionuclides, chemotherapeutic agents, and toxins.

12. A Myo/Nog cell targeting molecule for use in treating cancer according to claim 1, wherein said molecule is for administration directly to a tumor or the surrounding tissue.

13. An *in vitro* method of detecting an increased risk for cancer or metastatic potential in a subject, said method comprising detecting Myo/Nog cells in a biological sample from a subject, wherein an increase in Myo/Nog cells in the biological sample compared to a corresponding biological sample from a normal subject is indicative of an increased risk of cancer or metastatic potential in said subject.

14. The method of claim 12, wherein said method further comprises contacting the biological sample with at least one Myo/Nog cell targeting molecule conjugated to at least one detection molecule.

15. The method of claim 14, wherein said Myo/Nog cell targeting molecule is an antibody specific for the G8 antigen, a syndecan, or heparin sulfate proteoglycan.
